# EUROPÄISCHE PATENTSCHRIFT

(11) **EP 0 535 455 B1**
(45) Veröffentlichungstag und Bekanntmachung des Hinweises auf die Patenterteilung: **12.03.1997**
(21) Anmeldenummer: 92115903.4
(22) Anmeldetag: 17.09.1992
(51) Int. Cl.: C07D 201/04, C07D 201/06

(54) **Verfahren zur Herstellung von Caprolactam durch Beckmann'sche Umlagerung von Cyclohexanonoxim**
Process for the production of caprolactam by Beckmann rearrangement of cyclohexanone oxime
Procédé pour la production de caprolactame par réarrangement de Beckmann de cyclohexanone oxime

(30) Priorität: 30.09.1991 DE 4132498
(43) Veröffentlichungstag der Anmeldung: 07.04.1993
(73) Patentinhaber: BASF Aktiengesellschaft, 67063 Ludwigshafen (DE)
(72) Erfinder: Fuchs, Hugo, Dr., W-6700 Ludwigshafen (DE); Neubauer, Gerald, Dr., W-6940 Weinheim (DE); Ritz, Josef, Dr., W-6700 Ludwigshafen (DE)

(56) Entgegenhaltungen:
- EP-A- 0 271 014
- US-A- 3 914 217
- US-A- 3 953 438
- DATABASE WPI Week 7348, Derwent Publications Ltd., London, GB; AN 73-74124 & JP-B-700121691

## Beschreibung

Gegenstand der Erfindung ist ein Verfahren zur Herstellung von Caprolactam durch Beckmann'sche Umlagerung von Cyclohexanonoxim mit Oleum in mindestens zwei hintereinander schalteten Umlagerungsstufen.

Aus der japanischen Patentveröffentlichung 48/39949 ist ein Verfahren bekannt, bei dem man Cyclohexanonoxim mit Oleum in mehreren hintereinander geschalteten Reaktionszonen umsetzt, wobei man mindestens 70 % des Oleum der ersten Reaktionszone zuführt und Cyclohexanonoxim auf die einzelnen Reaktionszonen verteilt zugibt. Nach einem anderen in der US-PS 3 953 438 beschriebenen Verfahren wird Cyclohexanonoxim mit Oleum in zwei hintereinander geschalteten Umlaufzonen umgesetzt, wobei man den größeren Teil des Cyclohexanonoxims der ersten Reaktionszone und den kleineren Teil des Cyclohexanonoxims der zweiten Reaktionszone zugibt und die gesamte Oleummenge der ersten Reaktionszone zuführt. Mit den Verfahren nach dem Stand der Technik gelingt es zwar, die Permanganatabsorptionszahl wesentlich zu erniedrigen. Es hat sich jedoch herausgestellt, daß mit den steigenden Anforderungen an die Qualität des Caprolactams die so erhaltene Qualität nicht mehr den Anforderungen entspricht.

Aus der EP-A 271 014 ist auch schon ein Verfahren bekannt, bei dem man die Beckmann'sche Umlagerung in einem Kreislaufsystem durchführt und das erhaltene Reaktionsgemisch anschließend ohne weitere Zugabe von Cyclohexanonoxim in eine Nachverweilzone bei einer Temperatur von 95°C für 120 Minuten beläßt. Hierdurch gelingt es zwar, die Qualität weiter zu verbessern, jedoch der Verbrauch an Oleum konnte nicht gesenkt werden. Bei der Beckmann'schen Umlagerung von Cyclohexanonoxim zu Caprolactam ist man bestrebt, den Verbrauch an Säure so klein wie möglich zu halten, um den Zwangsanfall von Ammonsulfat zu minimieren. Je geringer jedoch das Verhältnis von Oleum zu Cyclohexanonoxim wird, um so größer ist die Gefahr einer Qualitätsverschlechterung insbesondere was UV-aktive Verbindungen wie Oktahydrophenazin betrifft.

Es war deshalb die technische Aufgabe gestellt, ein Verfahren zur Herstellung von Caprolactam durch Beckmann'sche Umlagerung von Cyclohexanonoxim mit Oleum zur Verfügung zu stellen, bei dem das so hergestellte Caprolactam nicht nur eine niedere Permanganatabsorptionszahl, eine niedere UV-Kennzahl sowie einen verminderten Gehalt an Oktahydrophenazin aufweist, sondern zugleich der Säureverbrauch so tief wie möglich gehalten wird.

Diese technische Aufgabe wird gelöst in einem Verfahren zur Herstellung von Caprolactam durch Beckmann'sche Umlagerung von Cyclohexanonoxim mit Oleum bei einer Temperatur von 85 bis 125°C in mehreren hintereinander geschalteten Umlagerungsstufen, wobei man einen Teil des die letzte Umlagerungsstufe verlassenden Reaktionsgemisches in mindestens eine der vorangehenden Umlagerungsstufen zurückführt.

Das neue Verfahren hat den Vorteil, daß auf einfache Weise die Qualität des erzeugten Caprolactams verbessert wird, wobei insbesondere ein niedriges Niveau für die Permanganattitrationszahl, die Werte für die UV-Kennzahl sowie den Gehalt an Oktahydrophenazin erzielt werden. Ferner hat das neue Verfahren den Vorteil, daß dies erzielt wird mit einem verminderten Verbrauch an Oleum, wodurch der Zwangsanfall an Ammoniumsulfat bei der Gewinnung des Caprolactams vermindert wird.

In der Regel geht man von schmelzflüssigem Cyclohexanonoxim, z.B. mit einer Temperatur von 80 bis 95°C aus. Das geschmolzene Cyclohexanonoxim hat in der Regel einen Wassergehalt von 1 bis 7 Gew.%, insbesondere einen Wassergehalt von 3,5 bis 6 Gew.%.

Die Umlagerung wird in mehreren hintereinander geschalteten Umlagerungsstufen, z.B. 2 bis 4 hintereinander geschalteten Umlagerungsstufen durchgeführt. Hierbei wird die Umsetzung in einem Gemisch aus Caprolactam und Oleum durchgeführt. Die Umlagerungsstufen können als Rührbehälter ausgeführt sein, vorteilhaft wird jedoch das Gemisch aus Caprolactam und Oleum in jeder Stufe im Kreislauf geführt. Über Verteilervorrichtungen werden geschmolzenes Cyclohexanonoxim und in getrennter Form über Verteilervorrichtungen Oleum zugeführt. Nach dem erfindungsgemäßen Verfahren gibt man in jeder Stufe Cyclohexanonoxim zu, vorteilhaft in von Stufe zu Stufe abnehmenden Mengen während mindestens 70 Gew.-%, insbesondere mindestens 90 Gew.-%, vorzugsweise die gesamte Menge an erforderlichem Oleum bereits der ersten Stufe zugeführt werden. Es hat sich bewährt, wenn man in der ersten Umlagerungsstufe 60 bis 85 Gew.-Teile Cyclohexanonoxim sowie das gesamte Oleum zugibt und die restliche Menge in abnehmendem Verhältnis den weiteren Umlagerungsstufen zuführt.

In den einzelnen Kreisläufen wird zweckmäßig die 40 bis 150fache Menge des Kreislaufvolumens je Stunde im Kreis geführt. Durch Kühlen wird eine Temperatur von 85 bis 125°C, insbesondere 95 bis 120°C, eingehalten. Es hat sich bewährt, wenn man die Temperatur von Stufe zu Stufe um 5 bis 15°C erhöht. Beispielsweise wird in einer ersten Stufe eine Temperatur von 95 bis 103°C eingehalten, in der zweiten Umlagerungsstufe eine Temperatur von 103 bis 110°C und in der dritten Umlagerungsstufe eine Temperatur von 110 bis 118°C.

In der Regel geht man von Oleum mit einem SO₃-Gehalt von 24 bis 34 Gew.% aus, vorteilhaft wird Oleum mit einem SO₃-Gehalt von 27 bis 32 Gew.% angewandt. Das Reaktionsgemisch in den einzelnen Stufen besteht im wesentlichen aus Caprolactam, Schwefelsäure, SO₃, abnehmenden Mengen an nicht umgesetztem Cyclohexanonoxim sowie geringen Mengen nicht näher definierten Nebenprodukten.

Vorteilhaft hält man bei einem dreistufigen Verfahren in der ersten Stufe eine Säurezahl von 115 bis 125, in der zweiten Stufe von 105 bis 112 und in der dritten Stufe von 102 bis 105 ein. Die Säurezahl, ausgedrückt als meq H₂SO₄ je 10 g Reaktionsgemisch, wurde durch Titration bestimmt.

In jeder Umlagerungsstufe wird vorteilhaft eine Verweilzeit von 10 bis 60 Minuten eingehalten.

Aus der letzten Umlagerungsstufe wird ein Gemisch, das im wesentlichen aus Caprolactam, Schwefelsäure, in geringen Menge SO₃ besteht, fortlaufend entnommen in dem Maße wie Cyclohexanonoxim und Oleum den vorangehenden Stufen zugeführt wurden.

Ein wesentliches Merkmal der Erfindung ist es, daß man einen Teil, z.B. 5 bis 40 Vol.%, des der letzten Stufe entnommenen Reaktionsgemisches in mindestens eine der vorhergehenden Umlagerungsstufen zurückführt. Besonders bewährt hat es sich bei Kreisläufen, wenn man 5 bis 35 Vol.%, insbesondere 10 bis 30 Vol.% des Kreislaufvolumens pro Stunde, d.h. der stündlich in der letzten Umlagerungsstufe im Kreis geführten Menge, der letzten Umlagerungsstufe in die vorangehende Stufen zurückführt. Vorteilhaft führt man den zurückzuführenden Teil des Reaktionsgemisches in die vorletzte Umlagerungsstufe zurück.

Caprolactam wird aus dem Umlagerungsgemisch z.B. wie folgt gewonnen: Das so erhaltene Reaktionsgemisch, das im wesentlichen aus Caprolactam, Schwefelsäure, restlichem Schwefeltrioxid und Nebenprodukten besteht, wird mit Ammoniak neutralisiert. Vorteilhaft wird das Reaktionsgemisch in einem Kreislaufsystem einer 35 bis 45 Gew.%igen wäßrigen Ammonsulfatlösung zugeführt, mit dieser vermischt und gasförmiges Ammoniak eingeleitet und bis zu einem pH-Wert von 4 bis 5 neutralisiert. Das sich nunmehr abscheidende Rohlactam wird von der gesättigten Ammonsulfatlösung z.B. durch Dekantieren abgetrennt und mit Benzol oder Toluol extrahiert. Nach Abtrennen des Extraktionsmittels wird das Caprolactam durch Destillation unter vermindertem Druck gereiningt.

Das nach dem erfindungsgemäßen Verfahren erhältliche Caprolactam zeichnet sich durch eine größere Reinheit aus und eignet sich zur Herstellung von Polycaprolactam für Faserzwecke. Das Verfahren nach der Erfindung sei an folgenden Beispielen veranschaulicht:

### Vergleichsbeispiel

### Dreistufige Umlagerung ohne Rückführung

In einem Kreislaufsystem mit ca. 5 l Inhalt, bestehend aus 3 Mischkreisen, Verbindungsleitungen, Pumpen, Kühler und einem Überlaufgefäß, wurde die ca. 150fache Menge des Kreislaufvolumens in jedem Mischkreis im Kreislauf geführt. Über eine Verteilervorrichtung wurde die zur Umlagerung benötigte Oleummenge in den ersten Mischkreis eingeführt. Die Oximmenge wurde im Gewichtsverhältnis 80 : 15 : 5 in den 1., 2. bzw. 3. Mischkreis eingebracht.

Das verwendete Cyclohexanonoxim hatte einen Wassergehalt von 4,2 Gew.%. Das zugeführte Oleum hatte einen freien SO₃-Gehalt von 32 %. Das Gewichtsverhältnis von Oleum zu Oximmenge betrug 0,93.

Die mittlere Verweilzeit im Kreislaufsystem betrug etwa 23 Minuten. Die Temperaturen in den einzelnen Mischkreisen betrugen 100, 105, 115°C. Der gemessene freie SO₃-Gehalt im Umlagerungsgemisch der 3. Stufe betrug 4,8 %.

Die in den dreistufigen Reaktionskreislauf eingebrachten Mengen treten als Reaktionsprodukt am Überlaufgefäß wieder aus dem Kreislauf aus und werden mit Ammoniak oder Ammoniakwasser bis zu einem pH-Wert von 4,6 neutralisiert. Das sich abscheidende Rohlactam wird abgetrennt und auf übliche Weise durch Extraktion und nachfolgender Vakuumdestillation vorgereinigt.

Im erhaltenen Extraktlactam wurden folgende Kennzahlen gefunden:

| | | |
|---|---|---|
| PTZ (Permanganattitrationszahl) | | 19 |
| Extinktion | 290 nm/10/50 | 6,1 |
| Oktahydrophenazin | ppm | 0,8 |
| Flüchtige Basen | meq/kg | 0,7 |
| Gaschromatographisch feststellbare Verunreinigungen | ppm | 373 |
| Restoximgehalt | ppm | 5 |

### Beispiel 1

### Arbeitsweise mit Rückführung, 3-stufige Umlagerung

Im gleichen Kreislaufsystem wie in Vergleichsbeispiel beschrieben, wird das Cyclohexanonoxim unter den dort angegebenen Bedingungen in 3 Stufen umgelagert.

Die Oleumzufuhr erfolgt in die 1. Stufe, während das Cyclohexanonoxim im Verhältnis 80 : 15 : 5 in den 1., 2. bzw. 3. Mischkreis zugeführt wird.

Ca. 20 % des Kreislaufvolumens pro Stunde der 3. Stufe wurde in die 2. Umlagerungsstufe zurückgeführt.

Der Gehalt an freiem SO₃ in dem Umlagerungsgemisch, das den 3. Mischkreis verläßt, betrug 4,9 %.

Die Säurezahl betrug in der ersten Stufe 118, in der zweiten Stufe 106 und in der dritte Stufe 104,5.

Das fertige Umlagerungsgemisch wird wie beschrieben aufgearbeitet. Im Extraktlactam werden folgende Kennzahlen erreicht:

| | | |
|---|---|---|
| PTZ | | 16 |
| Extinktion | 290 nm/10/50 | 5,2 |
| Oktahydrophenazin | ppm | 0,5 |
| Flüchtige Basen | meq/kg | 0,5 |
| Gaschromatographisch feststellbare Verunreinigungen | ppm | 133 |
| Restoximgehalt | ppm | <1 |

Zur Bestimmung der PTZ wurden 100 g Caprolactam bei 20°C in 250 ml 50 gew.-%iger Schwefelsäure gelöst und mit einer ¹/₁₀-n Kaliumpermanganat-Lösung so lange versetzt, bis sich ein stabiler rosa Farbton einstellte. Der Verbrauch in ml an Kaliumpermanganat-Lösung multipliziert mit 10 (d.h. bezogen auf 1 kg Caprolactam) entspricht der PTZ.

## Patentansprüche

1. Verfahren zur Herstellung von Caprolactam durch Beckmann'sche Umlagerung von Cyclohexanonoxim mit Oleum bei einer Temperatur von 85 bis 125°C in mehreren hintereinander geschalteten Umlagerungsstufen, dadurch gekennzeichnet, daß man einen Teil des die letzte Umlagerungsstufe verlassenden Reaktionsgemisches in mindestens eine der vorangehenden Umlagerungsstufen zurückführt.

2. Verfahren nach Anspruch 1, dadurch gekennzeichnet, daß man 2 bis 3 Umlagerungsstufen anwendet.

3. Verfahren nach den Ansprüchen 1 und 2, dadurch gekennzeichnet, daß man 10 bis 30 % des Kreislaufvolumens pro Stunde der letzten Umlagerungsstufe in mindestens eine der vorangehende Umlagerungsstufen zurückführt.

4. Verfahren nach den Ansprüchen 1 bis 3, dadurch gekennzeichnet, daß man Reaktionsgemisch aus der letzten Umlagerungsstufe in die unmittelbar vorangehende Umlagerungsstufe zurückführt.

## Claims

1. A process for preparing caprolactam by Beckmann rearrangement of cyclohexanone oxime with oleum at from 85 to 125°C in a plurality of rearrangement stages arranged in series, which comprises returning a portion of the reaction mixture leaving the last rearrangement stage to at least one of the preceding rearrangement stages.

2. A process as claimed in claim 1, wherein 2 or 3 rearrangement stages are applied.

3. A process as claimed in claim 1 or 2, wherein from 10 to 30% of the circulating volume per hour is returned from the last rearrangement stage to at least one of the preceding rearrangement stages.

4. A process as claimed in claim 1 or 2 or 3, wherein reaction mixture is returned from the last rearrangement stage to the immediately preceding rearrangement stage.

## Revendications

1. Procédé de production de caprolactame par réarrangement de Beckmann de cyclohexanone oxime avec de l'oléum à une température de 85 à 125 °C en plusieurs étapes de réarrangement successives, caractérisé en ce qu'on recycle une partie du mélange réactionnel sortant de la dernière étape de réarrangement dans au moins une des étapes de réarrangement précédentes.

2. Procédé selon la revendication 1, caractérisé en ce qu'on utilise deux à trois étapes de réarrangement.

3. Procédé selon les revendications 1 et 2, caractérisé en ce qu'on recycle 10 à 30 % du volume de circulation par heure de la dernière étape de réarrangement dans au moins une des étapes de réarrangement précédentes.

4. Procédé selon les revendications 1 à 3, caractérisé en ce qu'on recycle du mélange réactionnel de la dernière étape de réarrangement dans l'étape de réarrangement directement précédente.
